# EUROPÄISCHE PATENTSCHRIFT

(11) **EP 2 455 059 B1**
(45) Veröffentlichungstag und Bekanntmachung des Hinweises auf die Patenterteilung: **04.06.2014**
(21) Anmeldenummer: 10191957.9
(22) Anmeldetag: 19.11.2010
(51) Int. Cl.: A61K 6/083, C08F 4/32, C08L 33/26, C08L 33/10, C08L 33/08, A61K 6/00, C08F 2/48

(54) **Polymerisierbare Zusammensetzung mit verbesserter Durchhärtungstiefe**
Polymerisable compound having improved hardening depth
Composition polymérisable dotée d'une profondeur de durcissement améliorée

(43) Veröffentlichungstag der Anmeldung: 23.05.2012
(73) Patentinhaber: Ivoclar Vivadent AG, 9494 Schaan (LI)
(72) Erfinder: Moszner, Norbert, Prof. Dr., 9493, Mauren (LI); Salz, Ulrich, Dr., 88131, Lindau (DE); Burtscher, Peter, Dr., A-6830, Rankweil (AT); Liska, Robert, Prof. Dr., 2123, Schleinbach (AT); Gugg, Astrid, 1160, Wien (AT); Gorsche, Christian, 1140, Wien (AT)
(74) Vertreter: UEXKÜLL & STOLBERG

(56) Entgegenhaltungen:
- EP-A1- 2 058 340
- EP-A2- 0 787 750
- WO-A1-01/76536
- WO-A1-2010/067790
- DE-A1- 19 928 238
- JP-A- 2007 034 063
- SU-A1- 238 779
- JAMES A. WARREN: "Solution of a field theory model of frontal photopolymerization", PHYSICAL REVIEW, vol. E72, 3 August 2005 (2005-08-03), DOI: 10.1103/PhysRevE.72.021801

## Beschreibung

Die vorliegende Erfindung betrifft polymerisierbare Zusammensetzungen, die sich insbesondere als Dentalmaterialien eignen, wie Prothesenmaterialien, Materialien für Inlays, Onlays, Kronen oder Brücken sowie Füllungen.

Zur Aushärtung von polymerisierbaren Werkstoffen wie Dentalmaterialien finden unterschiedliche Polymerisationsmechanismen Anwendung. Bei Werkstoffen, die auf (Meth)acrylaten aufgebaut sind, erfolgt die Aushärtung über eine radikalische Polymerisation, wobei die Radikalbildung und damit die Aushärtung thermisch, chemisch, oder durch Licht initiiert werden kann. Die thermische Aushärtung findet vor allem bei Prothesenmaterialien Anwendung, kommt aber für eine Aushärtung unter oralen Bedingungen nicht in Frage. Die zur chemischen Initiierung verwendeten Redox-Intiator-Systeme bestehen mindestens aus zwei Komponenten, meist einem Peroxid und einem Reduktionsmittel. Aufgrund der hohen Reaktionsgeschwindigkeit von Peroxid und Reduktionsmittel dürfen beide Komponenten erst kurz vor der Aushärtung des Dentalmaterials zusammen gebracht werden, und es steht nur eine kurze Verarbeitungszeit zur Verfügung. Vor allem bei hochgefüllten Füllungskompositen ist darüber hinaus das Mischen der sogenannten Initiator-Paste mit der Beschleuniger-Paste, ohne dass dabei Luftblasen eingeschlossen werden, nur schwierig realisierbar, so dass hierfür hauptsächlich Photoinitiatoren eingesetzt werden. Nachteilig ist weiterhin die geringe Lagerstabilität von als Initiatoren verwendeten Peroxiden. Ein wesentlicher Nachteil von lichthärtenden Dentalmaterialien ist die beschränkte Durchhärtungstiefe, insbesondere von pigmentierten Kompositen, so dass deren Aushärtung schichtenweise erfolgen muss, was zeitaufwendig ist.

Bei der sogenannten Frontalpolymerisation (FP) wird eine Reaktionszone erzeugt, die von der Probenoberfläche durch die gesamte Reaktionsmischung wandert und so zu einem polymerisierten Produkt führt. A. Khan, J. A. Pojman, Trends in Polymer Science, 4 (1996) 253-257, beschreiben die Frontalpolymerisation von n-Butylacrylat, bei der die Reaktion oberflächlich durch Wärme gestartet wird. Die bei der Polymerisation der Oberflächenschicht entstehenden Reaktionswärme löst die Polymerisation in der angrenzenden Schicht aus, und es bildet sich so eine Polymerisationsfront, die mit einer Geschwindigkeit von etwa 1 cm pro Minute durch eine Polymerisationsmischung wandert. In der Reaktionszone werden Temperaturen von bis zu 290°C erreicht. Da die hohen Temperaturen zum Verdampfen der Monomeren und damit zur Blasenbildung führen können, wird die Reaktion unter einem Druck von > 15 x 10⁵ Pa durchgeführt. Gemäß Washington und Steinbock, Polymer News, 2003, Vol. 28, 303-310, können hohe Temperaturen zu einer Zersetzung des Initiators führen ("initiator burnout") und so die Polymerisationsfront stoppen.

Nason et al., Macromolecules 38 (2005) 5506-5512, offenbaren die UV-Licht-induzierte Frontalpolymerisation von multifunktionellen (Meth)acrylaten. An der Polymerisationsfront wurden Temperaturen von über 200°C gemessen, die für die intraorale dentale Anwendung nicht geeignet sind.

Die US-PS 4,222,835 offenbart durch Frontalpolymerisation härtbare Zusammensetzungen, die sich zur Beschichtung von Glasfasern oder anderen Objekten eignen sollen.

US-PS 6,057,406 und US-PS 6,313,237 offenbaren die Herstellung von polymeren Gradientenwerkstoffen durch thermisch initiierte Frontalpolymerisation.

Aus der US-PS 6,533,503 ist ein durch Frontalpolymerisation härtbarer Mörtel bekannt, der sich insbesondere zur Befestigung Ankerbolzen und Verstärkungseisen in Bohrlöchern eigenen soll.

DE 199 28 238 A1 offenbart polymerisierbare Dentalmassen, die mindestens ein bi- oder höherfunktionelles ethylenisch ungesättigtes Monomer, ein Redoxinitiatorsystem und Weichmacher enthalten. Das Redoxinitiatorsystem enthält ein Barbitursäurederivat und/oder Malonylsulfamid in Kombination mit einem ein- oder mehrfunktionellen Carbonsäureperoxyester. Die Zusammensetzungen sollen sich durch eine verbesserte Lagerstabilität auszeichnen.

WO 01/76536 offenbart zweikomponentige Dentalmassen mit niedriger Abbindetemperatur auf der Basis von ethylenisch ungesättigten Monomeren, die als Initiatorsystem ein Barbitursäurederivat und/oder Malonylsulfamid in Kombination mit einer Schwermetallverbindung als Beschleuniger enthalten.

EP 0 787 750 A2 betrifft strukturisomere Poly(alkylethylene) mit verbesserten Verarbeitungseigenschaften, die nach einem Bestrahlungsverfahren, Schmelzreaktionsverfahren oder Festphasenreaktionsverfahren durch Umsetzung von Poly(alkylethylenen) mit mono-, di- oder polyfunktionellen Monomeren, gegebenenfalls in Gegenwart von Peroxiden wie tert. Butylpermethacrylat, hergestellt werden können.

EP 2 058 340 A1 offenbart verzweigte Polypropylencopolymere, die durch Umsetzung von Polypropylencopolymeren mit Peroxiden wie tert.Butylpermethacrylat hergestellt werden können und die sich insbesondere für Extrusionsverfahren eignen sollen.

SU 238 779 betrifft die Herstellung von Latex durch Umsetzung von ungesättigten Perverbindungen wie tert. Butylpermethacrylat mit Vinylmonomeren oder einer Mischung aus Dien- und Vinylmonomeren in einem Wasseremulsionspolymerisationsverfahren. Die Verwendung ungesättigter Perverbindungen soll Seitenketten mit aktiven Peroxidgruppen ergeben, die das Vulkanisieren des Latex erleichtern sollen.

JP 2007 034063 A offenbart die Herstellung von Lichtleitern mit großem Brechungsindex zwischen der zentralen Achse und der Peripherie durch Frontalpolymerisation.

Die WO 2010/067790 A1 betrifft dual härtbare Materialkits, die ein radikalisch polymerisierbares Monomer, einen Photoinitiator und ein organisches Peroxid enthalten.

Der Erfindung liegt die Aufgabe zugrunde, Dentalwerkstoffe, mit großer Durchhärtungstiefe be-welche die obigen Nachteile nicht aufweisen und die insbesondere über eine ausreichend lange Verarbeitungszeit verfügen und die ohne Bildung hoher Temperaturen härten.

Diese Aufgabe wird erfindungsgemäß durch Zusammensetzungen gelöst, die neben einem polymerisierbaren Bindemittel mindestens einen Initiator gemäß der folgenden Formel I enthalten:

R¹-X-O-O-[Y-O-O-]ₙR² (Formel I)

in der
R¹ = H, ein linearer, verzweigter oder cyclischer aliphatischer C₁-C₂₀-Rest, der eine oder mehrere Mehrfachbindungen und/oder ein oder mehrere Heteroatome ausgewählt aus O, S und -NR⁵- enthalten kann, ein aromatischer C₆-C₁₄-Rest oder eine Kombination aus einem solchen aliphatischen und einem solchen aromatischen Rest ist oder eine der bei R² angegebenen Bedeutungen hat;
R⁵ = H, Phenyl oder C₁-C₉-Alkyl ist;
X = -CO- ist oder entfällt;
R² = -CO-M, -SO₂-M oder -P(=O)(-M)(-R³) ist;
R³ - R', -OR' oder -OO(CO)ₘR' ist, wobei R' H, ein linearer, verzweigter oder cyclischer aliphatischer C₁-C₂₀-Rest, der eine oder mehrere Mehrfachbindungen und/oder ein oder mehrere Heteroatome ausgewählt aus O, S und N enthalten kann, ein aromatischer C₆-C₁₄-Rest oder eine Kombination aus einem solchen aliphatischen und einem solchen aromatischen Rest ist, und m 0 oder 1 ist;
M = -CH=CH₂ oder -C(R⁴)=CH₂ ist;
R⁴ - ein C₁-C₁₀-Alkylrest oder ein C₃-C₈-Cycloalkylrest, wobei diese Reste ein oder mehrere S- oder O-Atome enthalten können, ein Phenylrest oder Benzylrest, wobei die genannten Reste halogeniert und insbesondere fluoriert oder perfluoriert sein können, -F, -Cl oder -Br ist;
Y = ein zweiwertiger, linearer, verzweigter oder cyclischer aliphatischer C₁-C₈-Rest ist; und
n = 0, 1 oder 2 ist.

Durch die Formel I werden nur solche Verbindungen erfasst, die mit der chemischen Valenzlehre vereinbar sind.

Bevorzugt sind solche Verbindungen gemäß Formel I, in der mindestens eine der Variablen eine der folgenden Bedeutungen hat:
R¹ - H, ein linearer, verzweigter oder cyclischer aliphatischer C₁-C₆-Rest, der eine Mehrfachbindung und/oder ein oder mehrere Heteroatome ausgewählt aus O und S enthalten kann, ein aromatischer C₆-C₁₀-Rest oder eine Kombination aus einem solchen aliphatischen und einem solchen aromatischen Rest oder eine der bei R² angegebenen Bedeutungen, vorzugsweise ein verzweigter aliphatischer C₃-C₆-Rest oder Cumyl-Rest;
X = -CO- oder entfällt, vorzugsweise entfällt;
R² - -CO-M, -SO₂-M oder -P(=O)(-M)(-R³), vorzugsweise -CO-M oder -P(=O)(-M)(-R³) ;
R³ - R', -OR' oder -OO(CO)ₘR', wobei R' H, ein linearer, verzweigter oder cyclischer aliphatischer C₁-C₆-Rest, ein aromatischer C₆-C₁₀-Rest oder eine Kombination aus einem solchen aliphatischen und einem solchen aromatischen Rest ist, und m 0 oder 1 ist, vorzugsweise R' oder OR', wobei R' ein linear oder verzweigter C₁-C₆-Rest ist;
M = -CH=CH₂ oder -C(R⁴)=CH₂, insbesondere -CH=CH₂;
R⁴ - ein C₁-C₂-Alkylrest, -F, -Cl, -Br, -CF₃ oder ein Phenylrest, vorzugsweise -CH₃, -CF₃ oder F;
Y = ein zweiwertiger, linearer, verzweigter oder cyclischer aliphatischer C₁-C₈-Rest, vorzugsweise eine verzweigter C₆-C₈-Rest; und
n = 0, 1 oder 2, vorzugsweise 0 oder 1.

Bei den Verbindungen der Formel (I) handelt es sich um polymerisationsfähige Peroxid-Initiatoren. Es wird angenommen, dass diese Verbindungen bei ihrer Polymerisation durch Anlagerung von Radikalen Peroxid-Spezies ergeben, die bei tieferer Temperatur zerfallen und damit eine Verringerung der Polymerisationsfronttemperatur ermöglichen. Dieser Vorgang wird hierin als radikal-induzierte Destabilisierung bezeichnet. Die Destabilisierung beruht wahrscheinlich auf der Erhöhung des Verzweigungsgrades. Durch Anlagerung eines Radikals an die Doppelbindung der Gruppe M entsteht ein Radikal, das ein weiteres Monomermolekül addieren kann und dadurch den Verzweigungsgrad am C-Atom neben der CO-Gruppe erhöht. Beispielsweise bildet sich bei der Anlagerung des Radikals • R an die Doppelbindung von tert.-Butylperacrylat und der weiteren Anlagerung eines Monomermoleküls Vinyl-Z an das gebildete Radikal eine stärker verzweigte Spezies (vgl. Struktur im gestrichelten Kasten), die der Struktur des Isobuttersäure-tert.-butylesters nahekommt:

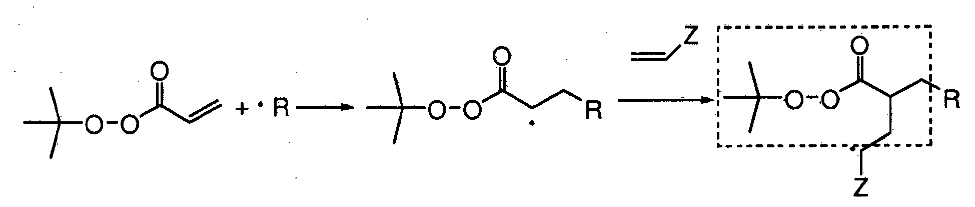

Diese Spezies ist aufgrund des erhöhten Verzweigungsgrades im Vergleich zum eingesetzten tert.-Butylperacrylat thermisch leichter spaltbar und gestattet somit eine Verringerung der Polymerisationsfronttemperatur. Die erfindungsgemäßen Zusammensetzungen zeichnen sich durch einen Gehalt an Initiatoren der Formel I aus und können daher bei relativ geringen Fronttemperaturen durch Frontalpolymerisation gehärtet werden. Dies hat den Vorteil, dass ein Sieden von Monomeren und damit eine Blasenbildung, die zu Inhomogenitäten und Instabilität führen kann, vermieden wird. Die Anwendung von Überdruck bei der Polymerisation ist nicht erforderlich. Durch die geringe Polymerisationsfronttemperatur wird zudem eine Zersetzung von Initiatoren verhindert und eine stabile Polymerisationsfront erzielt.

Die Initiatoren gemäß Formel I sind relativ einfach zugänglich. Die Synthese der Initiatoren kann z.B. durch Umsetzung von bekannten Hydroperoxiden mit ungesättigten Säurehalogeniden erfolgen.

Allgemein:

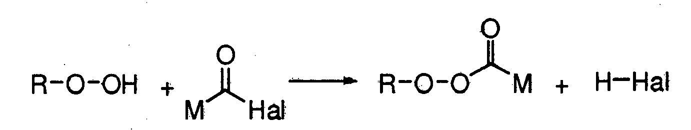

Konkretes Beispiel:

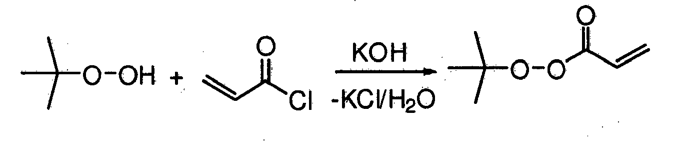

Wird dabei von Wasserstoffperoxid als Perverbindung ausgegangen, sind Initiatoren mit symmetrischer Struktur zugänglich: Konkretes Beispiel:

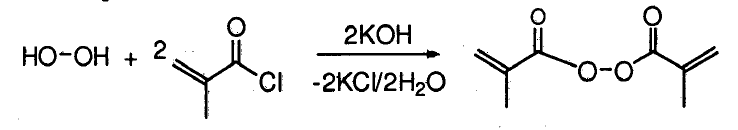

Bevorzugte Beispiele für die erfindungsgemäßen Initiatoren gemäß Formel I, sind insbesondere:

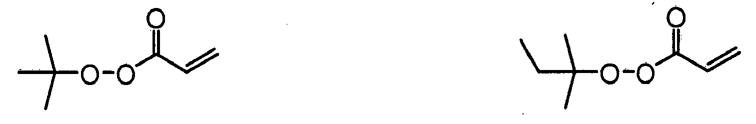

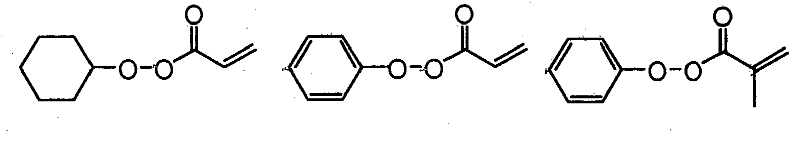

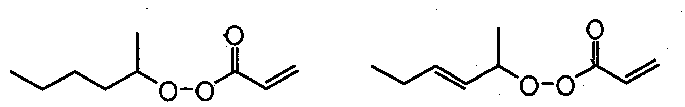

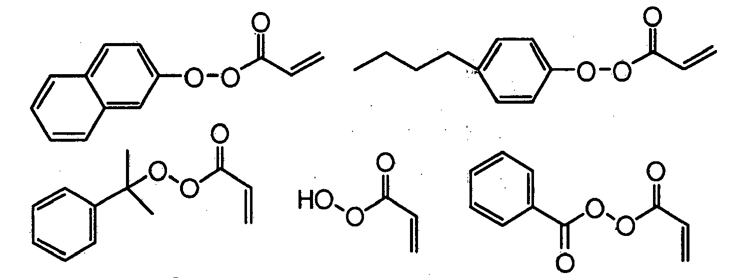

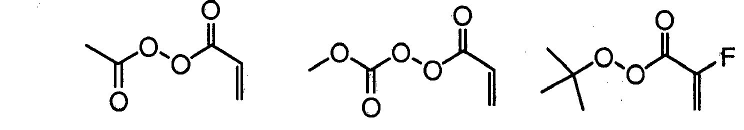

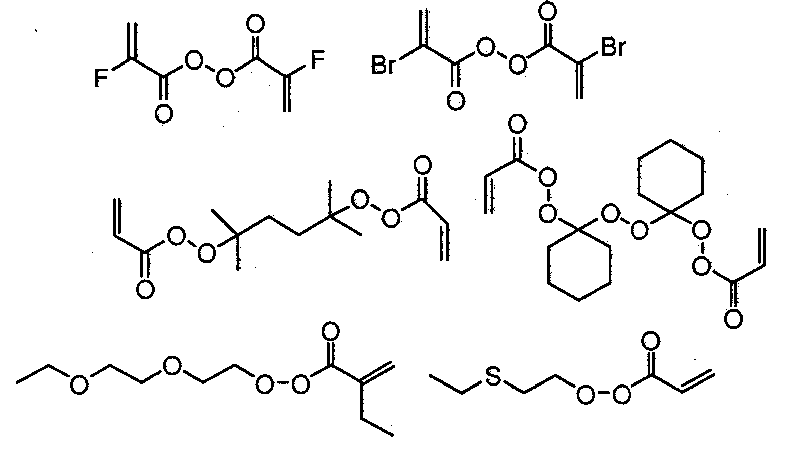

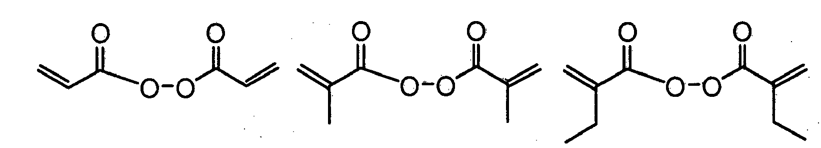

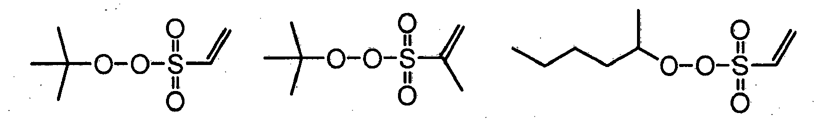

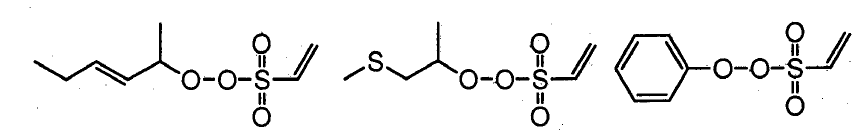

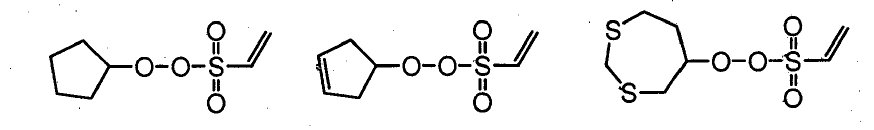

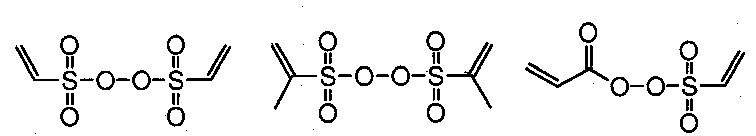

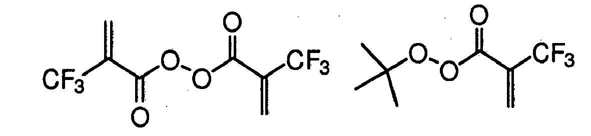

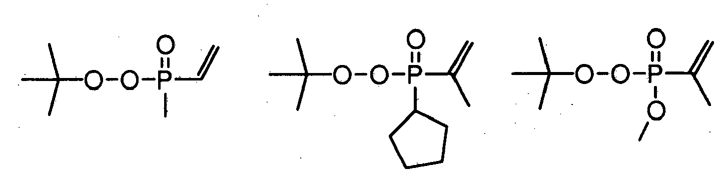

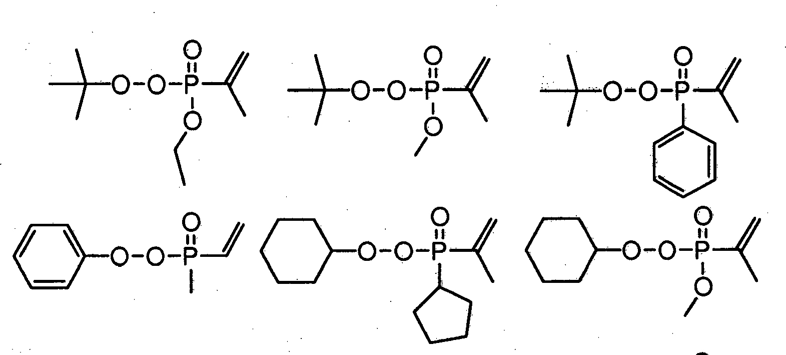

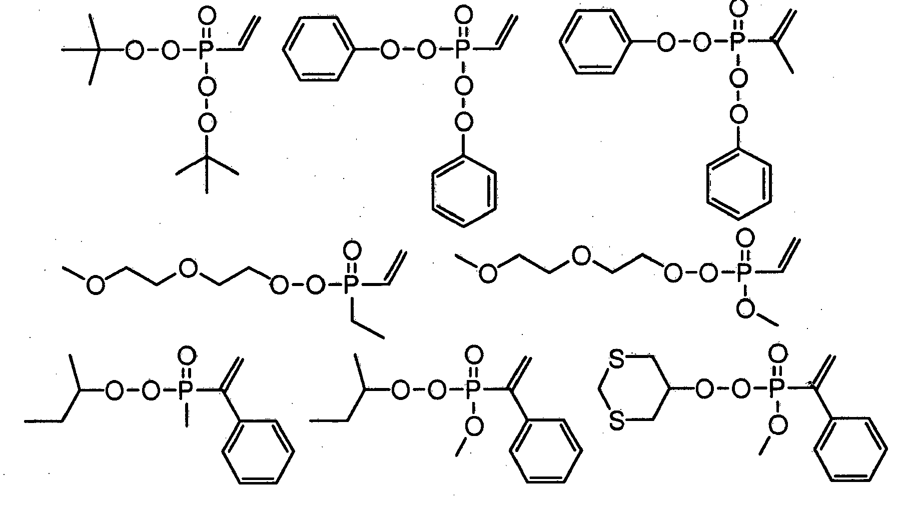

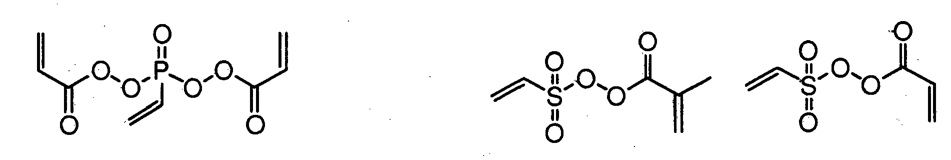

Die erfindungsgemäßen Zusammensetzungen eignen sich zur Anwendung als Dentalwerkstoff bzw. zur Herstellung von Dentalwerkstoffen. Hierzu enthalten die erfindungsgemäßen Zusammensetzungen ein polymerisierbares Bindemittel, das vorzugsweise aus Polyadditionsharzen und besonders bevorzugt aus radikalischen Polymerisationsharzen ausgewählt ist. Das polymerisierbare Bindemittel wird im folgenden auch als Polyreaktionsharz bezeichnet, wobei Polyreaktion der Oberbegriff für Polymerisation und Polyaddition ist.

Als Polyreaktionsharze lassen sich bekannte Polymerisations- und Polyadditionsharze verwenden, die in der Regel aus einer Mischung von niedermolekularen oder oligomeren Monomeren bestehen, welche eine oder mehrere polyreaktionsfähige Gruppen enthalten. Im Falle von Polymerisationsharzen kommen insbesondere radikalisch polymerisationsfähige Harze in Frage. Bei den Polyadditionsharzen sind vor allem Thiol-En-Harze geeignet. Sowohl im Fall der Polymerisations- als auch der Polyadditionsharze ist die Verwendung von vernetzenden Polyreaktionsharzen bevorzugt.

Die Verwendung von radikalisch polymerisierbaren Harzen als Bindemittel ist erfindungsgemäß besonders bevorzugt. Ganz besonders bevorzugt sind Zusammensetzungen, die als radikalisch polymerisierbares Bindemittel ein mono- oder multifunktionelles (Meth)acrylat, ein N-mono- oder N-disubstituiertes (Meth)-acrylamid oder eine Mischung davon enthalten. Dabei sind weiterhin solche radikalisch polymerisierbaren Bindemittel bevorzugt, die mindestens 50 Gew.-%, besonders bevorzugt mindestens 60 Gew.-% und insbesondere mindestens 70 Gew.-%, d.h. 70 bis 100 Gew.-% polyfunktionelle (Meth)acrylate und/oder polyfunktionelle (Meth)acrylamide enthalten, wobei sich die Prozentangaben hier auf die Masse des polymerisierbaren Bindemittels beziehen. Unter monofunktionellen (Meth)acrylaten bzw. (Meth)acrylamiden werden Verbindungen mit einer, unter polyfunktionellen (Meth)acrylaten bzw. (Meth)acrylamiden Verbindungen mit zwei oder mehr, vorzugsweise 2 bis 4 (Meth)acrylat- bzw. (Meth)acrylamidgruppen verstanden. Polyfunktionelle Monomere werden auch als vernetzende Monomere bezeichnet.

Bevorzugte mono- und multifunktionelle (Meth)acrylate sind Methyl-, Ethyl-, Hydroxyethyl-, Hydroxypropyl-, Butyl-, Benzyl-, 2-Phenoxyethyl-, Glycidyl-, Tetrahydrofurfuryl- oder Isobornyl(meth)acrylat, Bisphenol-A-di(meth)acrylat, Bis-GMA (ein Additionsprodukt aus 2 Mol Methacrylsäure und 1 Mol Bisphenol-A-diglycidylether), Additionsprodukte aus 2 Mol 2-Hydroxyalkyl(meth)acrylat und 1 Mol Diisocyanaten, wie z.B. Hexamethylendiisocyanat oder 2,2,4-Trimethylhexamethylendiisocyanat, Di-, Tri- oder Tetraethylenglycoldi(meth)acrylat, (meth)acrylat-terminierte Poly(ethylenglycol)e und Poly(propylenglycol)e, Trimethylolpropantri(meth)acrylat, Pentaerythrittetra(meth)acrylat, sowie Glycerindi- und -tri(meth)acrylat, 1,4-Butandioldi(meth)acrylat, 1,10-Decandioldi(meth)acrylat 1,12-Dodecandioldi(meth)acrylat und Dipentaerythritmonohydroxypenta(meth)acrylat. Außerdem sind auch ethoxylierte Monomere, wie z.B. ethoxyliertes Bis-GMA oder ethoxyliertes Trimethylolpropantri(meth)acrylat geeignet. Dabei zeigen Acrylate im Vergleich zu den entsprechenden Methacrylaten eine höhere Reaktivität, was sich in einer schnelleren Polymerisationsfront bemerkbar macht. Weiterhin geeignete radikalisch polymerisierbare Monomere sind N-mono- oder -disubstitituierte (Meth)acrylamide, wie z.B. N-Ethylacrylamid, N,N-Dimethacrylamid, N-(2-Hydroxyethyl)acrylamid, N-Methyl-N-(2-hydroxyethyl)acrylamid, N-Ethylmethacrylamid oder N-(2-Hydroxyethyl)methacrylamid. Außerdem können vinyl- oder allylgruppenhaltige Monomere wie N-Vinylpyrrolidon oder Allylether als radikalisch polymerisierbare Bindemittel verwendet werden. Weiterhin einsetzbar sind entsprechende Vernetzermonomere, z.B. vernetzende Pyrrolidone, wie z.B. 1,6-Bis(3-vinyl-2-pyrrolidonyl)-hexan, oder kommerziell zugängliche Bisacrylamide, wie Methylen- oder Ethylenbisacrylamid, Bismethacrylamide, wie z.B. N,N'-Diethyl-1,3-bis(acrylamido)-propan, 1,3-Bis(methacrylamido)-propan, 1,4-Bis(acrylamido)-butan oder 1,4-Bis-(acryloyl)-piperazin.

Gemäß einer weiteren Ausführungsform der Erfindung sind Zusammensetzungen bevorzugt, die als Bindemittel ein Polyadditionsharz verwenden. Als Polyadditionsharz bevorzugt sind vor allem Thiol-En-Harze, die aus Mischungen von di- oder multifunktionellen Mercaptoverbindungen und di- oder multifunktionellen ungesättigten Monomeren, vor allem Allyl- oder Norbonenverbindungen bestehen. Di- oder multifunktionell bedeuten, dass die jeweiligen Verbindungen 2 oder mehrere, bevorzugt 2, 3 oder 4, reaktive Allyl-, Norbornenyl oder Mercaptogruppen enthalten. Auch hier sind solche Bindemittel bevorzugt, die mindestens 50 Gew.-%, besonders bevorzugt mindestens 60 Gew.-% und insbesondere mindestens 70 Gew.-%, d.h. 70 bis 100 Gew.-% multifunktionelle Verbindungen enthalten, wobei sich die Prozentangaben hier auf die Masse des polymerisierbaren Bindemittels beziehen.

Beispiele für mono- oder multifunktionellen Mercaptoverbindungen sind o-, m- bzw. p-Dimercaptobenzol und Ester der Thioglykol- oder der 3-Mercaptopropionsäure mit Ethylen-, Propylen- oder Butylenglykol, Hexandiol, Glycerin, Trimethylolpropan oder Pentaerythrit. Beispiele für di- oder multifunktionelle Allylverbindungen sind Ester von Allylalkohol mit Di- oder Tricarbonsäuren, wie Malon-, Malein- Glutar-, Bernstein-, Adipin-, Sebacin-, Phthal-, Terephthal- oder Gallussäure sowie mono- oder trifunktionelle Allylether, wie z.B. Diallylether, α ,ω-Bis[allyloxy]alkane, Resorcin- oder Hydrochinondiallylether sowie Pyrogalloltriallylether, oder andere Verbindungen wie z.B. 1,3,5-Triallyl-1,3,5-triazin-2,4,6-(1H,3H,5H)-trion, Tetraallylsilan oder Tetraallylorthosilikat. Beispiele für di- oder multifunktionelle Norbonenverbindungen sind Diels-Alder-Additionsprodukte von Cyclopentadien oder Furan mit di- oder multifunktionellen (Meth)acrylaten, sowie Ester bzw. Urethane von 5-Norbornen-2-methanol oder 5-Norbornen-2-ol mit Di- oder Polycarbonsäuren, wie z.B. Malon- Malein-, Glutar-, Bernstein-, Adipin-, Sebacin-, Phthal-, Terephthal- oder Gallussäure, bzw. mit Di- oder Polyisocyanaten, wie Hexamethylendiisocyanat bzw. dessen cyclisches Trimer, 2,2,4-Trimethylhexamethylendiisocyanat, Toluylendiisocyanat oder Isophorondiisocyanat.

Außerdem lassen sich auch di- oder multifunktionelle Alkine mit den Thiol-Komponenten umsetzen (Thiol-In-Harze).

Neben den Initiatoren der Formel I enthalten die erfindungsgemäßen Dentalwerkstoffe vorzugsweise mindestens einen weiteren Initiator für die radikalische Polymerisation und/oder für die Polyaddition. Als Initiatoren für die radikalische Polymerisation sind thermische Initiatoren und insbesondere Photoinitiatoren bevorzugt. Gemäß einer besonders bevorzugten Ausführungsform betrifft die Erfindung daher Zusammensetzungen, die neben einem Initiator der Formel (I) radikalisch polymerisierbares Bindemittel und einen Photoinitiator für die radikalische Polymerisation enthalten.

Als thermische Initiatoren lassen sich vor allem organische Perverbindungen und Azo-Verbindungen einsetzen. Als organische Perverbindungen eignen sich Hydroperoxide, wie Cumolhydroperoxid oder tert.-Butylhydroperoxid, Dialkylperoxide, z.B. Ditert.-butylperoxid oder Dicumolperoxid, Diperoxiketale, wie 1,1-Di(tert.-butylperoxi)cyclohexan oder Methylethylketonperoxid, Persäureester, wie tert.-Amylperoxiacetat, tert.-Butylperpivalat, tert.-Butylper-2-ethylhexanoat oder tert.-Butylperisobutyrat, Diacylperoxide, wie Dibenzoylperoxid, Diisobutyrylperoxid, Dioctanoylperoxid, oder Dilauroylperoxid, und Peroxidicarbonate, wie z.B. Diisopropylperoxidicarbonat, Di-n-butylperoxidicarbonat oder Dicyclohexylperoxidicarbonat. Beispiele für geeignete Azoverbindungen sind 2,2'-Azobisisobutyronitril, Dimethyl-2,2'-azobisisobutyrat, 1,1'-Azobis(1-cyclohexannitril), 2,2'-Azobis(2,4,4-trimethylpentan) oder Azobis(2,4-dimethylvaleronitril).

Die Menge an gasbildenden Perverbindungen wie z.B. Nitrilen und Peroxiden beträgt vorzugsweise weniger als 2 Gew.-%, besonders bevorzugt weniger als 1 Gew.-%. Azoverbindungen werden vorzugsweise in einer Menge von weniger als 1 Gew.-%, besonders bevorzugt weniger als 0,5 Gew.-% eingesetzt. Perverbindungen, die beim thermischen Zerfall kein Gas bilden wie z.B. Persulfate, lassen sich auch bis zu 5 Gew.-% einsetzen. Alle Prozentangaben beziehen sich auf die Gesamtmasse des Werkstoffs.

Bevorzugte Photoinitiatoren für Zusammensetzungen auf der Basis von radikalisch polymerisierbaren Harzen sowie von Thiol-En-Polyadditionsharzen sind die bekannten radikalischen UV-Photoinitiatoren vom Norrish-Typ-I (vgl. J.P. Fouassier, Photoinitiation, Photopolymerization and Photocuring, Hanser Publishers, Munich, Vienna, New York 1995, 22 ff.), wie Benzilketale, Benzoinether, Methylthiophenylmorpholinoketone, Dialkoxyacetophenone, Hydroxyalkylphenylacetophenone, Aminoketone oder Acylphosphinoxide. Geeignete bimolekulare UV-Photoinitiatoren vom Norrish-Typ II sind die Systeme Benzophenon/Amine, Michler's Keton/Benzophenon und Thioxanthon/Amine (vgl. J.P. Fouassier, J.F. Rabek (Hrsg.), Radiation Curing in Polymer Science and Technology, Vol. III, Elsevier Applied Science, London und New York 1993, 189ff.). Geeignete Photoinitiatoren für den sichtbaren Bereich (vgl. J.P. Fouassier, J.F. Rabek (Hrsg.), Radiation Curing in Polymer Science and Technology, Vol. III, Elsevier Applied Science, London und New York 1993, 228ff.) sind z.B. Bisacylphosphinoxide, aber vor allem α-Diketone wie 9,10-Phenanthrenchinon, Diacetyl, Furil, Anisil, 4,4'-Dichlorbenzil und 4,4'-Dialkoxybenzil und besonders Campherchinon. Zur Beschleunigung der Initiierung mittels α-Diketonen werden Kombinationen mit aromatischen Aminen bevorzugt verwendet. Redoxsysteme, die sich bereits bewährt haben, sind Kombinationen aus Campherchinon mit Aminen, wie N,N-Dimethyl-p-toluidin, N,N-Dihydroxyethyl-p-toluidin, 4-Dimethylaminobenzoesäureethylester oder strukturverwandte Systeme. Besonders geeignet sind Norrish-Typ-I-Photoinitiatoren vor allem Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium. Dabei lassen sich auch Mischungen der verschiedenen Photoinitiatoren einsetzen, wie z.B. Dibenzoyldiethylgermanium in Kombination mit Campherchinon und 4-Dimethylaminobenzoesäureethylester.

Zur Verhinderung einer vorzeitigen Polyreaktion enthalten die erfindungsgemäßen Zusammensetzungen vorzugweise einen oder mehrere Polymerisations- bzw. Polyadditionsinhibitoren als Stabilisatoren, die eine Lagerstabilität der Materialien über ca. 2-3 Jahre ermöglichen und eine unkontrollierte Polyreaktion verhindern können. Für radikalische Reaktionsharze und Thiol-En-Harze werden als sogenannte aerobe Inhibitoren Phenole, wie Hydrochinonmonomethylether (MEHQ) oder 2,6-Di-tert.-butyl-4-methyl-phenol (BHT), eingesetzt die nur in Gegenwart von Sauerstoff effektiv wirksam sind und vorzugsweise in einem Konzentrationsbereich von 200-2000 ppm verwendet werden. Anaerobe Inhibitoren, wie z.B. Phenothiazin, 2,2,6,6-Tetramethylpiperindin-1-oxyl-Radikal (TEMPO), Iod, Kupfer-(I)-iodid, wirken dagegen in geringsten Konzentrationen (10-50 ppm) auch bei Abwesenheit von Sauerstoff. Eine Polymerisation findet erst dann statt, wenn diese Additive verbraucht sind. Dabei ist es auch häufig von Vorteil, eine Mischung von aeroben und anaeroben Inhibitoren einzusetzen.

Weiterhin können die Polyreaktionsharze vorzugsweise auch radikalisch polymerisierbare, säuregruppenhaltige Haftmonomere enthalten. Geeignete säuregruppenhaltige Monomere sind polymerisationsfähige Carbonsäuren, wie Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin und 4-Vinylbenzoesäure. Beispiele für geeignete Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxabutyl]-acrylsäure oder 2-[4-(Dihydroxyphosphoryl)-2-oxabutyl]-acrylsäureethyl- oder -2,4,6-trimethylphenylester. Beispiele für geeignete acide polymerisationsfähige Phosphorsäureester sind 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloylpiperidin-4-yl)-ester, 6-(Methacrylamido)hexyldihydrogenphosphat und 1,3-Bis-(N-acryloyl-N-propyl-amino)-propan-2-yldihydrogenphosphat. Beispiele für geeignete polymerisationsfähige Sulfonsäuren sind Vinylsulfonsäure, 4-Vinylphenylsulfonsäure oder 3-(Methacrylamido)propylsulfonsäure.

Die aufgezählten aciden Monomere sind radikalisch polymerisierbar und eignen sich daher insbesondere zur Kombination mit radikalisch polymerisierbaren Bindemitteln. Bevorzugte Monomere sind 4-(Meth)acryloyloxyethyltrimellitsäureanhydrid, 10-Methacryloyloxydecylmalonsäure, 2-Methacrylamidoethylphosphonsäure, 2-[4-(Dihydroxyphosphoryl)-2-oxa-butyl]-acrylsäureethyl- oder -2,4,6-trimethylphenylester, 10-Methacryloyloxydecyl-dihydrogenphosphat, 6-(Methacrylamido)hexyldihydrogenphosphat oder 1,3-Bis-(N-acryloyl-N-propyl-amino)-propan-2-yldihydrogenphosphat. Acide Monomere und insbesondere die bevorzugten aciden Monomere werden vorzugsweise in einer Menge von 0 bis zu 20 Gew.-%, besonders bevorzugt von 1 bis 10 eingesetzt, bezogen auf die Gesamtmasse des Dentalwerkstoffs.

Außerdem können die erfindungsgemäßen Zusammensetzungen beispielsweise zur Verbesserung der mechanischen Eigenschaften oder zur Einstellung der Viskosität mit organischen oder anorganischen Partikeln gefüllt werden. Die Füllstoffe haben vorzugsweise eine mittlere Partikelgröße von 0,01 bis 10 µm, besonders bevorzugt von 0,02 bis 5 µm. Bevorzugte anorganische partikuläre Füllstoffe sind amorphe kugelförmige Materialien auf der Basis von Oxiden, wie pyrogener Kieselsäure oder Fällungskieselsäure, ZrO₂ und TiO₂ bzw. Mischoxiden aus SiO₂, ZrO₂ und/oder TiO₂ mit einem mittleren Partikeldurchmesser von 10 bis 200 nm, Minifüllstoffe, wie Quarz-, Glaskeramik- oder Glaspulver mit einer durchschnittlichen Teilchengröße von 0,01 bis 1 µm, sowie röntgenopake Füllstoffe, wie Ytterbiumtrifluorid oder nanopartikuläres Tantal(V)-oxid oder Bariumsulfat, die vorzugsweise eine mittlere Partikelgröße von 10 bis 200 nm aufweisen.

Außerdem können die erfindungsgemäßen Zusammensetzungen weitere Additive enthalten, vor allem Lösungsmittel oder entsprechende Lösungsmittelgemische sowie Aromastoffe, Farbmittel, mikrobiocide Wirkstoffe, fluoridionenabgebende Additive, optische Aufheller, Weichmacher oder UV-Absorber.

Besonders bevorzugte erfindungsgemäße Zusammensetzungen zur Anwendung als Dentalmaterial enthalten die folgenden Komponeten:
a) 0,1 bis 10 Gew.-%, bevorzugt 0,2 bis 8 Gew.-% und besonders bevorzugt 0,5 bis 5 Gew.-% Initiator gemäß Formel I,
b) 0,01 bis 10 Gew.-%, besonders bevorzugt 0,1 bis 3,0 Gew.-% an einem oder mehreren thermischen Initiatoren und/oder Photoinitiatoren, vorzugsweise Photoinitiatoren,
c) 10 bis 99 Gew.-%, bevorzugt 20 bis 99 Gew.-% und besonders bevorzugt 25 bis 98 Gew.-% Bindemittel,
d) 0 bis 85 Gew.-%, besonders bevorzugt 0 bis 75 Gew.-%, ganz besonders bevorzugt 0 bis 60 Gew.-% und insbesondere 0 bis 40 Gew.-% an einem oder mehreren Füllstoffen,
e) 0 bis 30 Gew.-%, bevorzugt 0 bis 20 Gew.-% und besonders bevorzugt 0 bis 10 Gew.-% an Stabilisatoren und/oder Additiven.

Aufgrund der unterschiedlichen Reaktivität der radikalisch polymerisierbaren Monomeren und Polyadditionsharze wird der Initiator gemäß Formel (I) in Abhängigkeit vom verwendeten Bindemittel dabei vorzugsweise in folgenden Mengen eingesetzt:

| | | | | |
|---|---|---|---|---|
| Acrylat(e): | 0,2-5 Gew.-%, | besonders | bevorzugt | 0,5-2 Gew.-%; |
| Acrylamid(e): | 0,2-5 Gew.-%, | besonders | bevorzugt | 0,5-2 Gew.-%; |
| Methacrylat(e): | 1-10 Gew.-%, | besonders | bevorzugt | 2-5 Gew.-%; |
| Thiol-En-Harz(e): | 1-10 Gew.-%, | besonders | bevorzugt | 2-5 Gew.-%. |

Alle Gew.-Prozentangaben beziehen sich, wenn nicht anders angegeben, auf das Gesamtgewicht der Zusammensetzung. Erfindungsgemäß bevorzugt sind einkomponentige Zusammensetzungen.

Die Menge an Initiator der Formel (I) und ggf. weiteren thermischen Initiatoren variiert auch mit den Menge an Füllstoffen, Lösungsmittel und anderen nicht-reaktiven Komponenten. Ist ein hoher Anteil an nicht-reaktiven Komponenten vorhanden, wird eher eine hohe Initiatormenge gewählt, ist der Anteil an nicht-reaktiven Komponenten gering, reichen geringere Initiatormengen aus, um eine Durchhärtung der Materialien zu bewirken.

Die erfindungsgemäßen Zusammensetzungen zeichnen sich aufgrund ihres Gehalts an einem oder mehreren Initiatoren der Formel (I) dadurch aus, dass sie sich durch Frontalpolymerisation mit verringerter Fronttemperatur härten lassen, so dass die mit hohen Temperaturen einhergehenden Probleme vermieden werden. Die Menge an Initiator der Formel (I) wird vorzugsweise so gewählt, dass die Fronttemperatur zwischen 40 und 100°C, bevorzugt zwischen 50 und 80°C liegt.

Die erfindungsgemäßen Zusammensetzungen eignen sich als Dentalwerkstoffe, insbesondere als Prothesenkunststoffe, Zemente, Komposite, Kompositzemente oder Füllungskomposite, Verblendmaterialien und Inlaymaterialien.

Die Erfindung betrifft auch Verfahren zur Herstellung von Formkörpern, die dentaler Natur sind. Hierzu wird eine erfindungsgemäße Zusammensetzung zunächst in die gewünschte Form gebracht, beispielsweise durch das Einbringen in eine geeignete Gussform, und dann durch gehärtet, vorzugsweise durch Frontalpolymerisation. Beispielsweise sind moderne Prothesenkunststoffe heutzutage oft lichthärtende Materialien, die aufgrund ihrer Schichtdicke gewisse Durchhärtungsprobleme zeigen. Solche Prothesen werden nach der Formgebung z.B. in einem sogenannten Lichtofen gehärtet, d.h. sie werden mit Licht einer geeigneten Wellenlänge bestrahlt und hierdurch die Polymerisationsreaktion ausgelöst. Hier können die erfindungsgemäßen Dentalwerkstoffe vorteilhaft eingesetzt werden, da durch die Anwendung der Frontalpolymerisation die Durchhärtungstiefe verbessert werden kann.

Ein weiterer Gegenstand der Erfindung ist die Verwendung von Initiatoren der Formel (I) zur Verringerung der Fronttemperatur und/oder zur Verbesserung der Durchhärtungstiefe bei der Frontalpolymerisation, insbesondere bei der Frontalpolymerisation der oben definierten Zusammensetzungen auf der Basis radikalisch polymerisierbarer Bindemittel. Die Verwendung der oben definierten Verbindungen der Formel (I) als Initiatoren für die Frontalpolymerisation ist ebenfalls Gegenstand der Erfindung.

Außerdem betrifft die Erfindung die Verwendung von durch Frontpolymerisation härtbaren Zusammensetzungen als Dentalwerkstoff bzw. zur Herstellung eines Dentalwerkstoffs. Bevorzugt ist die Verwendung radikalisch polymerisierbarer Zusammensetzungen. Diese Zusammensetzungen enthalten vorzugsweise die oben angegebenen Komponenten, insbesondere in den definierten Mengen.

Die Verwendung der Frontalpolymerisation zur Härtung von Dentalmaterialien ist ebenfalls Gegenstand der Erfindung.

Die Erfindung wird im Folgenden anhand von Beispielen näher erläutert.

### Ausführungsbeispiele

### Beispiel 1: Synthese von tert.-Butylperacrylat (tBPA)

3,9 mmol tert.-Butylhydroperoxid (als 70%ige wässrige Lösung) wurden in einem Rundkolben vorgelegt und ca. 10.000 ppm MeHQ als Inhibitor, gelöst in ca. 3 ml H₂O, zugegeben. Unter Eisbadkühlung wurde 1 Äquivalent KOH (50%, aq.) langsam zugetropft, danach wurden 12 Äquivalente NaCl als 20 %ige Lösung zugeführt. Nach 0,5 h Rühren und Abkühlen auf -5° bis 0°C wurden 1,1 Äquivalente Acrylsäurechlorid (enthält 400 ppm Phenothiazin als Inhibitor) sehr langsam unter starkem Rühren zugetropft, nach weiteren 3,5 h Rühren bei gleichbleibender Temperatur wurden ca. 15 ml Methylenchlorid zugegeben und die Reaktionslösung konnte mit NaHCO₃ -Lösung extrahiert werden. Der organischen Phase wurden nach Trocknen über wasserfreiem Na₂SO₄ und Filtrieren abermals ca. 10.000 ppm Hydrochinonmonomethylether (MEHQ) als Inhibitor zugeführt. Mittels Flash-Säulenchromatographie (CH₂Cl₂) konnte das Produkt gereinigt werden. Da der Inhibitor bei der Reinigung abgetrennt wurde, wurden nochmals 1.000 ppm MeHQ zugegeben. Durch behutsames Absaugen konnte das Lösungsmittel ohne größere Produktverluste entfernt werden und wurden ca. 200 mg (33 % Ausbeute) des tert.-Butylperacrylat als gelbliche Flüssigkeit erhalten.
¹H-NMR (CDCl₃), δ (ppm): 6.54-6.44 (dd, 1H, C=CH₂); 6.18-6.04 (m, 1H, CH=CH₂); 5.93-5.87 (dd, 1H, C=CH₂); 1.34 (s, 9H, - C(CH₃)₃) .
¹³C-NMR (CDCl₃), δ (ppm) : 164.3 (C=O) ; 132.0 (-CH=CH₂) ; 124.3 (-CH=CH₂) ; 83.8 (-C(CH₃)₃); 26.2 (-C(CH₃)₃).

### Beispiel 2: Thermische Stabilität von tert.-Butylperacrylat

Mittels DSC wurde die thermische Stabilität von Hexandioldimethacrylat (HDDMA)-Mischungen verschiedener Perverbindungen untersucht, die 167 µmol/g der Per-Verbindung enthielten. Dabei wurde mit einer Aufheizrate von 1 °C/min auf 200 °C erwärmt, um zu bestimmen, bei welcher Temperatur ausreichend Peroxid zerfällt, um eine Polymerisation zu initiieren (vgl. Tab. 1). Die Einwaage pro DSC-Schälchen betrug ca. 15 mg, als "Vergleichsprobe" wurde ein Schälchen mit Al₂O₃ verwendet. Als Perverbindung wurden neben dem *tert.*-Butylperacrylat (tBPA) aus Beispiel 1, Propionsäure- (tBPP) und Isobuttersäure-tert.-butylperester (tBPiB) untersucht. Die Initiierungstemperatur von reinem HDDMA wurde ebenfalls ermittelt. Die Ergebnisse sind in Tabelle 1 zusammengefasst.

Tabelle 1 zeigt, dass die Zugabe von tBPA zu HDDMA die Starttemperatur der Polymerisation deutlich verringert (ΔT ≈ 60°), d.h. dass tBPA als Initiator aktiv ist.

**Tab. 1: Starttemperatur der Polymerisation von HDDMA initiiert durch verschiedene Perverbindungen**

| **Perverbindung** | **Starttemperatur (°C)** |
|---|---|
| keine Vergleichsbeispiel *) | 163 |
| Isobuttersäure-tert.-butylperester (tBPiB)*) | 79,5 |
| Propionsäure-tert.-butylperester (tBPP)*) | 98,5 |
| *tert*.-Butylperacrylat (tBPA) | 103,5 |

| | |
|---|---|
| *) Vergleichsbeispiel | |

Die Ergebnisse zeigen weiter eine tiefere Starttemperatur des Isobuttersäureperesters tBPiB, der eine Modelsubstanz für das einpolymerisierte Peracrylat tBPA darstellt und eine Destabilisierung des eingesetzten Peracrylats durch die radikalinduzierte Polymerisation belegt. Das weniger verzweigte tBPP weist eine höhere Starttemperatur als tBPiB auf. Aus den Ergebnissen kann geschlossen werden, dass bei tBPA die Frontalpolymerisation mit einer Polymerisationsfronttemperatur von etwa 80°C stattfindet.

### Beispiel 3: Lagerstabilität von tert.-Butylperacrylat

Um die Lagerstabilität des *tert*.-Butylperacrylat (tBPA) beurteilen zu können, wurden Formulierungen von N-Acryloylmorpholin (NAM) mit 1 µmol/g Bis(4-methoxybenzoyl)diethylgermanium als Photoinitiator und variierenden Mengen an tBPA (ca. 0.1% bzw. 1%, entsprechend 4,71 mmol/g bzw. 47,1mmol/g) bei 43°C über 8 Wochen gelagert und anschließend die Viskosität der Mischung bestimmt. Die Ergebnisse sind in Tabelle 2 gezeigt. Nach 8 Wochen ergab sich im Rahmen der Messgenauigkeit die gleiche Viskosität, was die gute Lagerstabilität des tBPA bei Raumtemperatur belegt. Es wurde keine Polymerisation beobachtet.

Die Ergebnisse belegen eine hohe Lagerstabilität von tert.-Butylperacrylat-haltigen Harzen bei Raumtemperatur.

**Tabelle 2: Viskosität tBPA in NAM, Scherrate 102 s⁻¹; Lagerzeit 8 Wochen**

| **Menge an tBPA [%]** | η **[mPa·s]** |
|---|---|
| - | 11,0 |
| 0,1% | 10,9 |
| 1% | 22,2 |

### Beispiel 4: Frontalpolymerisation (FP) von Harzmischungen

In zugeschmolzenen Pasteurpipetten wurden Bestrahlungsversuche mit Mischungen von Trimethylolpropantriacrylat (TMPTA) mit dem UV-Photoinitiator (5% Darocur 1173) und unterschiedlichen Mengen an tert.-Butylperacrylat (tBPA) unter Breitbandbestrahlung (5 sec, 320-500 nm, Lichtintensität = 3 W/cm²) durchgeführt. Die für die Messungen eingefüllten 450 mg der Formulierungen entsprachen einem Füllstand von ca. 14 mm. Die Pipette wurde von der Unterseite her bestrahlt, und es wurde die Dicke der polymerisierten Schicht gemessen. Die Ergebnisse sind in Tabelle 3 gezeigt.

Acrylsäure-tert.-butylester wurde als Vergleichsmonomer verwendet. Es enthält keine reaktive Peroxidgruppe und löst damit auch keine Frontalpolymerisation aus. Die Dicke der polymerisierten Schicht ist identisch zu der Schichtdicke, die ohne Zusatz erzielt wurde. Der Zusatz von 1 Gew.-% tBPA führte zu einer deutliche Zunahme der Schichtdicke, was ein Beleg dafür ist, dass hier eine Frontalpolymerisation stattfindet. Zur genauen Bestimmung der Schichtdicke wurde der Versuch mit einer größeren Menge an polymerisierbarer Zusammensetzung wiederholt. Es wurde eine Schichtdicke von 52 mm gemessen, was einer Verzehnfachung gegenüber dem Vergleichsbeispiel ist. Auch die Zugabe von nur 0,2 Gew.-% tBPA führte schon zu einer deutlichen Zunahme der Schichtdicke (ca. 60-70 %).

**Tabelle 3: Ergebnisse der FP in TMPTA**

| **Zusatz [%]** | **Schichtdicke (mm)** |
|---|---|
| - * | 4-5 |
| Acrylsäure-*tert*.-butylester 1%* | 4-5 |
| tBPA 1% | 13** |
| tBPA 0,2% | 7-8 |
| tBPA 1% | 52*** |
| 25% OX-50* | 4-5 |
| 25% OX-50 + tBPA 1%** | 12 |

| | |
|---|---|
| * Vergleichsbeispiel ** Gesamtes Monomer ausgehärtet *** 1 g Füllmenge | |

In einer zweiten Versuchsreihe wurde eine Mischung verwendet, die zusätzlich 25 Gew.-% Füllstoff enthielt (Aerosil OX-50, Fa. Degussa, SiO₂ mit einer Primärpartikelgröße von 40 nm). Dabei ergab sich mit 1% tBPA eine ausgehärtete Schicht von 12 mm und ohne tBPA nur 4-5 mm.

Die Ergebnisse belegen eine Frontalpolymerisation mit Harzsystemen, die eine ausreichende Menge des Peracrylats tBPA enthalten, wobei auch gefüllte Systeme eine Frontalpolymerisation ergeben. Mit solchen Rezepturen können beispielsweise Zahnprothesen durch Frontalpolymerisation hergestellt werden.

## Patentansprüche

1. Polymerisierbare Zusammensetzung, **dadurch gekennzeichnet, dass** sie
(i) einen Initiator gemäß der folgenden Formel I:
R¹-X-O-O-[Y-O-O-]ₙR² (Formel I)
in der
R¹ = H, ein linearer, verzweigter oder cyclischer aliphatischer C₁-C₂₀-Rest, der eine oder mehrere Mehrfachbindungen und/oder ein oder mehrere Heteroatome ausgewählt aus O, S und -NR⁵-enthalten kann, ein aromatischer C₆-C₁₄-Rest oder eine Kombination aus einem solchen aliphatischen und einem solchen aromatischen Rest ist oder eine der bei R² angegebenen Bedeutungen hat;
R⁵ = H, Phenyl oder C₁-C₉-Alkyl ist;
X = -CO- ist oder entfällt;
R² = -CO-M, -SO₂-M oder -P(=O) (-M) (-R³) ist;
R³ = R', -OR' oder -OO(CO)ₘR' ist, wobei R' H, ein linearer, verzweigter oder cyclischer aliphatischer C₁-C₂₀-Rest, der eine oder mehrere Mehrfachbindungen und/oder ein oder mehrere Heteroatome ausgewählt aus O, S und N enthalten kann, ein aromatischer C₆-C₁₄-Rest oder eine Kombination aus einem solchen aliphatischen und einem solchen aromatischen Rest ist, und m 0 oder 1 ist;
M = -CH=CH₂ oder -C(R⁴)=CH₂ ist;
R⁴ = ein C₁-C₁₀-Alkylrest oder ein C₃-C₈-Cycloalkylrest, wobei diese Reste ein oder mehrere S- oder O-Atome enthalten können, ein Phenylrest oder Benzylrest, wobei die genannten Reste halogeniert sein können, -F, -Cl oder -Br ist;
Y = ein zweiwertiger, linearer, verzweigter oder cyclischer aliphatischer C₁-C₈-Rest ist; und
n = 0, 1 oder 2 ist, und
(ii) polymerisierbares Bindemittel enthält,
zur Verwendung als Dentalmaterial für Füllungen oder als dentaler Zement.

2. Zusammensetzung nach Anspruch 1, in dem mindestens eine der Variablen der Formel I eine der folgenden Bedeutungen hat:
R¹ = H, ein linearer, verzweigter oder cyclischer aliphatischer C₁-C₆-Rest, der eine Mehrfachbindung und/oder ein oder mehrere Heteroatome ausgewählt aus O und S enthalten kann, ein aromatischer C₆-C₁₀-Rest oder eine Kombination aus einem solchen aliphatischen und einem solchen aromatischen Rest oder eine der bei R² angegebenen Bedeutungen;
X = -CO- oder entfällt;
R² = -CO-M, -SO₂-M oder -P(=O)(-M)(-R³);
R³ = R', -OR' oder -OO(CO)ₘR', wobei R' H, ein linearer, verzweigter oder cyclischer aliphatischer C₁-C₆-Rest, ein aromatischer C₆-C₁₀-Rest oder eine Kombination aus einem solchen aliphatischen und einem solchen aromatischen Rest ist, und m 0 oder 1 ist;
M = -CH=CH₂ oder -C(R⁴)=CH₂;
R⁴ = ein C₁-C₂-Alkylrest, -F, -Cl, -Br, -CF₃ oder ein Phenylrest;
Y = ein zweiwertiger, linearer, verzweigter oder cyclischer aliphatischer C₁-C₈-Rest; und
n = 0, 1 oder 2.

3. Zusammensetzung nach einem der vorhergehenden Ansprüche, die ein radikalisch polymerisierbares Bindemittel enthält, das aus mono- oder multifunktionellen (Meth)acrylaten, N-mono- oder N-disubstituierten (Meth)acrylamiden oder Mischung davon ausgewählt ist.

4. Zusammensetzung nach Anspruch 1 oder 2, die als Bindemittel eine Mischung von mono- und/oder multifunktionellen Mercaptoverbindungen und di- und/oder multifunktionellen ungesättigten Monomeren (Thiol-En-Harz) enthält.

5. Zusammensetzung nach einem der vorhergehenden Ansprüche, die zusätzlich mindestens einen Photoinitiator enthält.

6. Zusammensetzung nach einem der vorhergehenden Ansprüche, die mindestens einen Stabilisator enthält.

7. Zusammensetzung nach einem der vorhergehenden Ansprüche, die zusätzlich Füllstoff enthält.

8. Zusammensetzung nach einem der vorhergehenden Ansprüche, die zusätzlich mindestens ein Additiv enthält, das aus Lösungsmitteln, Aromastoffen, Farbmitteln, mikrobiociden Wirkstoffen, fluoridionenabgebenden Additiven, optischen Aufhellern, Weichmacher und UV-Absorber ausgewählt ist.

9. Zusammensetzung nach einem der vorhergehenden Ansprüche, die
(a) 0,1 bis 10 Gew.-% Initiator gemäß Formel I,
(b) 0,01 bis 10 Gew.-% Photoinitiator,
(c) 10 bis 99 Gew.-% polymerisierbares Bindemittel,
(d) 0 bis 85 Gew.-% Füllstoff
(e) 0 bis 30 Gew.-% Additiv enthält, jeweils bezogen auf die Gesamtmasse der Zusammensetzung.

10. Zusammensetzung nach Anspruch 9, die
Acrylat(e) als Bindemittel und 0,2-5 Gew.-% Initiator der Formel (I) oder
Acrylamid(e) als Bindemittel und 0,2-5 Gew.-% Initiator der Formel (I) oder
Methacrylat(e) als Bindemittel und 1-10 Gew-% Initiator der Formel (I) oder
Thiol-En-Harz(e) als Bindemittel und 1-10 Gew.-% Initiator der Formel (I) enthält.

11. Verfahren zur Herstellung von dentalen Formkörpern, bei dem man eine Zusammensetzung gemäß einem der Ansprüche 1 bis 10 durch Frontalpolymerisation härtet.

12. Verwendung von Verbindungen der Formel (I) als Initiator für die Frontalpolymerisation von Dentalwerkstoff.

13. Verwendung einer Zusammensetzung wie in einem der Ansprüche 1 bis 10 definiert zur Herstellung eines Dentalwerkstoffs.

14. Verwendung nach Anspruch 13 zur Herstellung eines Prothesenmaterials, Materials für Inlays, Onlays, Kronen oder Brücken, Komposits, Verblendmaterials oder Inlaymaterials.

## Claims

1. Polymerisable composition, **characterised in that** it comprises
(i) an initiator according to the following Formula I:
R¹-X-O-O-[Y-O-O-]ₙR² (Formula I)
in which
R¹ = H, a linear, branched or cyclic aliphatic C₁-C₂₀ group that can comprise one or more multiple bonds and/or one or more heteroatoms selected from O, S and -NR⁵-, an aromatic C₆-C₁₄ group or a combination of one such aliphatic and one such aromatic group or has one of the meanings given for R²;
R⁵ = H, phenyl or C₁-C₉ alkyl;
X = -CO- or is absent;
R² = -CO-M, -SO₂-M or -P(=O)(-M)(-R³);
R³ = R', -OR' or -OO(CO)ₘR', wherein R' is H, a linear, branched or cyclic aliphatic C₁-C₂₀ group that can comprise one or more multiple bonds and/or one or more heteroatoms selected from O, S and N, an aromatic C₆-C₁₄ group or a combination of such an aliphatic and such an aromatic group, and m is 0 or 1;
M = -CH=CH₂ or -C(R⁴)=CH₂;
R⁴ = a C₁-C₁₀ alkyl group or a C₃-C₈ cycloalkyl group, wherein these groups can comprise one or more S or O atoms, a phenyl group or benzyl group, wherein the cited groups can be halogenated, -F, -Cl or -Br;
Y = a divalent, linear, branched or cyclic aliphatic C₁-C₈ group; and
n = 0, 1, or 2, and
(ii) polymerisable binder,
for use as a dental material for fillings or as a dental cement.

2. Composition according to claim 1, in which at least one of the variables of Formula I has one of the following meanings:
R¹ = H, a linear, branched or cyclic aliphatic C₁-C₆ group that can comprise a multiple bond and/or one or more heteroatoms selected from O and S, an aromatic C₆-C₁₀ group or a combination of one such aliphatic and one such aromatic group or has one of the meanings given for R²;
X = -CO- or is absent;
R² = -CO-M, -SO₂-M or -P(=O)(-M)(-R³);
R³ = R', -OR' or -OO(CO)ₘR', wherein R' is H, a linear, branched or cyclic aliphatic C₁-C₆ group, an aromatic C₆-C₁₀ group or a combination of one such aliphatic and one such aromatic group, and m is 0 or 1;
M = -CH=CH₂ or -C(R⁴)=CH₂;
R⁴ = a C₁-C₂ alkyl group, -F, -Cl, -Br, -CF₃ or a phenyl group;
Y = a divalent, linear, branched or cyclic aliphatic C₁-C₈ group; and
n = 0, 1 or 2.

3. Composition according to one of the preceding claims comprising a radically polymerisable binder that is selected from monofunctional or multifunctional (meth)acrylates, *N*-monosubstituted or *N*-disubstituted (meth)acrylamides or a mixture thereof.

4. Composition according to claim 1 or 2 comprising a mixture of monofunctional and/or multifunctional mercapto compounds and difunctional and/or multifunctional unsaturated monomers (thiol-ene resin) as the binder.

5. Composition according to one of the preceding claims additionally comprising at least one photoinitiator.

6. Composition according to one of the preceding claims additionally comprising at least one stabiliser.

7. Composition according to one of the preceding claims additionally comprising filler.

8. Composition according to one of the preceding claims additionally comprising at least one additive that is selected from solvents, flavourings, dyes, microbiocidal active ingredients, fluoride ion-releasing additives, optical brighteners, plasticisers and UV absorbers.

9. Composition according to one of the preceding claims comprising
(a) 0.1 to 10 wt % initiator according to Formula I,
(b) 0.01 to 10 wt % photoinitiator,
(c) 10 to 99 wt % polymerisable binder,
(d) 0 to 85 wt % filler
(e) 0 to 30 wt % additive, relative in each case to the total mass of the composition.

10. Composition according to claim 9 comprising acrylate(s) as the binder and 0.2-5 wt % initiator of Formula (I) or acrylamide(s) as the binder and 0.2-5 wt % initiator of Formula (I) or methacrylate(s) as the binder and 1-10 wt % initiator of Formula (I) or thiol-ene resin(s) as the binder and 1-10 wt % initiator of Formula (I).

11. Process for producing dental moulded bodies, in which process a composition according to one of claims 1 to 10 is cured by frontal polymerisation.

12. Use of compounds of Formula (I) as the initiator for the frontal polymerisation of dental material.

13. Use of a composition as defined in one of claims 1 to 10 for producing a dental material.

14. Use according to claim 13 for producing a prosthetic material, material for inlays, onlays, crowns or bridges, composites, veneering material or inlay material.

## Revendications

1. Composition polymérisable, **caractérisée en ce qu'**elle contient :
i) un amorceur de formule I suivante :
R¹-X-O-O-[Y-O-O-]ₙR² (formule I)
dans laquelle
- R¹ représente un atome d'hydrogène, un groupe aliphatique en C₁-C₂₀, à chaîne linéaire, ramifiée ou cyclique, qui peut comporter une ou plusieurs liaison(s) multiple(s) et/ou un ou plusieurs chaînons hétéroatomique(s) choisi(s) parmi ceux qui sont symbolisés par -O-, -S- ou -NR⁵-, un groupe aromatique en C₆-C₁₄, ou une combinaison d'un tel groupe aliphatique et d'un tel groupe aromatique, ou bien a l'une des significations indiquées pour R² ;
- R⁵ représente un atome d'hydrogène, un groupe phényle ou un groupe alkyle en C₁-C₉ ;
- X représente un chaînon symbolisé par -CO-, ou ne représente rien ;
- R² représente une entité symbolisée par -CO-M, par -SO₂M ou par -P(=O)(-M)(-R³) ;
- R³ représente une entité symbolisée par R', par -OR' ou par -OO(CO)ₘR', où R' représente un atome d'hydrogène, un groupe aliphatique en C₁-C₂₀, à chaîne linéaire, ramifiée ou cyclique, qui peut comporter une ou plusieurs liaison(s) multiple(s) et/ou un ou plusieurs hétéroatome(s) choisi(s) parmi les atomes d'oxygène, de soufre et d'azote, un groupe aromatique en C₆-C₁₄, ou une combinaison d'un tel groupe aliphatique et d'un tel groupe aromatique, et l'indice m vaut 0 ou 1 ;
- M représente un groupe de formule -CH=CH₂ ou -C(R⁴)=CH₂ ;
- R⁴ représente un groupe alkyle en C₁-C₁₀ ou un groupe cycloalkyle en C₃-C₈, lesquels groupes peuvent comporter un ou plusieurs atome(s) de soufre ou d'oxygène, ou un groupe phényle ou benzyle, lesdits groupes pouvant être halogénés, ou bien représente un atome de fluor, de chlore ou de brome ;
- Y représente un groupe aliphatique divalent en C₁-C₈, à chaîne linéaire, ramifiée ou cyclique ;
- et l'indice n vaut 0, 1 ou 2 ;
ii) et un liant polymérisable,
pour utilisation en tant que matériau dentaire pour obturations ou en tant que ciment dentaire.

2. Composition conforme à la revendication 1, dans laquelle au moins l'un des symboles de la formule I a l'une des significations suivantes :
- R¹ représente un atome d'hydrogène, un groupe aliphatique en C₁-C₆, à chaîne linéaire, ramifiée ou cyclique, qui peut comporter une liaison multiple et/ou un ou plusieurs hétéroatome(s) choisi(s) parmi les atomes d'oxygène ou de soufre, un groupe aromatique en C₆-C₁₀, ou une combinaison d'un tel groupe aliphatique et d'un tel groupe aromatique, ou bien a l'une des significations indiquées pour R² ;
- X représente un chaînon symbolisé par -CO-, ou ne représente rien ;
- R² représente une entité symbolisée par -CO-M, par -SO₂M ou par -P(=O)(-M)(-R³) ;
- R³ représente une entité symbolisée par R', par -OR' ou par -OO(CO)ₘR', où R' représente un atome d'hydrogène, un groupe aliphatique en C₁-C₆, à chaîne linéaire, ramifiée ou cyclique, un groupe aromatique en C₆-C₁₀, ou une combinaison d'un tel groupe aliphatique et d'un tel groupe aromatique, et l'indice m vaut 0 ou 1 ;
- M représente un groupe de formule -CH=CH₂ ou -C(R⁴)=CH₂ ;
- R⁴ représente un groupe alkyle en C₁-C₂, un atome de fluor, de chlore ou de brome, un groupe trifluorométhyle, ou un groupe phényle ;
- Y représente un groupe aliphatique divalent en C₁-C₈, à chaîne linéaire, ramifiée ou cyclique ;
- et l'indice n vaut 0, 1 ou 2.

3. Composition conforme à l'une des revendications précédentes, qui contient un liant polymérisable par voie radicalaire, lequel est choisi parmi les acrylates et méthacrylates monofonctionnels ou polyfonctionnels, les acrylamides et méthacrylamides où l'atome d'azote porte un ou deux substituant(s), et les mélanges de tels composés.

4. Composition conforme à la revendication 1 ou 2, qui contient en tant que liant un mélange de thiols monofonctionnels et/ou polyfonctionnels et de monomères insaturés difonctionnels et/ou polyfonctionnels (résine thiol-alcène).

5. Composition conforme à l'une des revendications précédentes, qui contient en outre au moins un photo-amorceur.

6. Composition conforme à l'une des revendications précédentes, qui contient au moins un stabilisant.

7. Composition conforme à l'une des revendications précédentes, qui contient en outre une charge.

8. Composition conforme à l'une des revendications précédentes, qui contient en outre au moins un adjuvant, lequel est choisi parmi les solvants, arômes, colorants, agents microbicides, adjuvants libérant des ions fluorure, azurants optiques, assouplissants et agents absorbant un rayonnement ultraviolet.

9. Composition conforme à l'une des revendications précédentes, qui contient
a) de 0,1 à 10 % en poids d'amorceur de formule I,
b) de 0,01 à 10 % en poids de photo-amorceur,
c) de 10 à 99 % en poids de liant polymérisable,
d) de 0 à 85 % en poids de charge,
e) et de 0 à 30 % en poids d'adjuvant,
dans chaque cas par rapport au poids total de la composition.

10. Composition conforme à la revendication 9, qui contient :
- un ou des acrylate(s), en tant que liant, et de 0,2 à 5 % en poids d'amorceur de formule I,
- ou un ou des acrylamide(s), en tant que liant, et de 0,2 à 5 % en poids d'amorceur de formule I,
- ou un ou des méthacrylate(s), en tant que liant, et de 1 à 10 % en poids d'amorceur de formule I,
- ou une ou des résine(s) thiol-alcène, en tant que liant, et de 1 à 10 % en poids d'amorceur de formule I.

11. Procédé de préparation d'une pièce façonnée dentaire, dans lequel on fait durcir par polymérisation frontale une composition conforme à l'une des revendications 1 à 10.

12. Utilisation de composés de formule I en tant qu'amorceurs de polymérisation frontale d'un matériau dentaire.

13. Utilisation d'une composition conforme à l'une des revendications 1 à 10 pour la préparation d'un matériau dentaire.

14. Utilisation conforme à la revendication 13, pour la préparation d'un matériau de prothèse, de matériaux pour incrustations « in-lay » ou « on-lay », couronnes ou bridges, d'un matériau composite, d'un matériau de revêtement ou d'un matériau d'incrustation « in-lay ».
